# FASCICULE DE BREVET EUROPEEN

(11) **EP 2 488 490 B1**
(45) Date de publication et mention de la délivrance du brevet: **19.03.2014**
(21) Numéro de dépôt: 10785467.1
(22) Date de dépôt: 13.10.2010
(51) Int. Cl.: C07C 323/52, C08G 18/38

(54) **NOUVEAU PROCÉDÉ DE PRÉPARATION DE POLYOLS PAR THIOLISATION ET PRODUITS TELS QU'OBTENUS**
NEUARTIGES VERFAHREN ZUR HERSTELLUNG VON POLYOLEN DURCH THIOLIERUNG UND AUF DIESE WEISE GEWONNENE PRODUKTE
NOVEL METHOD FOR PREPARING POLYOLS BY MEANS OF THIOLATION AND PRODUCTS SUCH AS THOSE OBTAINED

(30) Priorité: 13.10.2009 FR 0957146
(43) Date de publication de la demande: 22.08.2012
(73) Titulaire: Centre National de la Recherche Scientifique CNRS, 75016 Paris (FR)
(72) Inventeur: CRAMAIL, Henri, F-33350 Sainte Terre (FR); BOYER, Aurélie, F-33000 Bordeaux (FR); CLOUTET, Eric, F-33880 Saint Caprais De Bordeaux (FR); ALFOS, carine, F-33600 Pessac (FR)
(74) Mandataire: Blot, Philippe Robert Emile
(86) Numéro de dépôt international: PCT/FR2010/052171
(87) Numéro de publication internationale: WO 2011/045536

(56) Documents cités:
- US-A- 5 380 886
- US-A- 6 107 433
- US-A1- 2005 154 221
- US-A1- 2007 232 816
- C.M. BUESS, ET AL.: "Addition of mercaptoacetic acid to terpenes and related compounds", JOURNAL OF ORGANIC CHEMISTRY, vol. 22, no. 2, février 1957 (1957-02), pages 197-200, XP002585110, AMERICAN CHEMICAL SOCIETY, WASHINGTON, DC, US ISSN: 0022-3263, DOI: 10.1021/jo01353a029
- N. WEBER, ET AL.: "Antioxidants eliminate stereomutation and thioether formation during lipase-catalysed thioesterification and transthioesterification for the preparation of uniform cis- and trans-unsaturated thioesters", CHEMISTRY AND PHYSICS OF LIPIDS, vol. 105, no. 2, avril 2000 (2000-04), pages 215-223, XP002585111, ELSEVIER SCIENCE IRELAND, LIMERICK, IE ISSN: 0009-3084, DOI: 10.1016/S0009-3084(00)00125-0

## Description

La présente invention concerne un nouveau procédé de polyols, notamment de diols, ainsi que les nouveaux polyols tels qu'obtenus.

Il existe différentes approches pour la synthèse de polymères à partir d'huiles végétales. La première, la plus répandue, consiste à considérer les triglycérides comme matériaux de base, ceux-ci pouvant être époxydés puis par exemple alcoolisés ou hydroformylés, afin de les rendre fonctionnels et polymérisables.

Une huile est un mélange de triglycérides (triesters) formés par condensation des acides gras et du glycérol. Le nombre élevé de types d'acides gras (jusqu'à 24) présents dans chaque corps gras et les multiples possibilités de leurs combinaisons avec les molécules de glycérol font que les corps gras sont des mélanges très complexes de composés dont les propriétés varient d'une huile à une autre. La nature des triglycérides peut donc varier au sein d'une même huile.

Les sites réactifs présents dans un triglycéride sont principalement les doubles liaisons et les fonctions ester. La réactivité des doubles liaisons permet d'introduire des fonctions hydroxyles, permettant ainsi l'accès à des monomères plurihydroxylés. Il est néanmoins impossible d'obtenir des triglycérides ayant des structures et des fonctionnalités parfaitement définies.

La synthèse de polyols issus d'huile végétale est bien décrite dans la littérature car ces derniers constituent d'excellents précurseurs pour la synthèse de polymères. Ces matériaux gagnent en popularité en raison de l'origine naturelle des précurseurs et des propriétés attractives apportées par la structure et la composition des huiles végétales. Les sites réactifs dans tous les corps gras sont les fonctions esters et les doubles liaisons. Certaines huiles possèdent également d'autres groupements comme des hydroxyles ou des époxydes.

Les doubles liaisons de ces composés ne sont généralement pas assez réactives pour servir de sites de polymérisation radicalaire. Néanmoins, à haute température (330°C), les doubles liaisons peuvent migrer le long du squelette pour former des sites conjugués, ce qui facilite les condensations de type Diels-Alder. Des oligomères ont été synthétisés par vulcanisation d'huiles avec du monochlorure de soufre et utilisés comme additifs dans l'industrie des gommes par exemple. Des oligomères ont également été synthétisés par polymérisation cationique en présence de trifluorure de bore (Croston et al., J. Amer. Oil Chem. Soc. 1952, 331-333), pour des applications dans des formulations d'encres. D'autres réactions faisant intervenir les doubles liaisons, comme la polymérisation par métathèse, ont permis d'obtenir des oligomères (Refvik et al., J.Amer. Oil Chem. Soc. 1999, 76, 93-98) et les matériaux qui en sont issus sont rarement exploitables car très mal définis.

Il est donc nécessaire de mieux contrôler la fonctionnalisation des huiles végétales.

Comme déjà indiqué, la présence de doubles liaisons sur le squelette permet l'introduction de groupements hydroxyles. Celle-ci peut être réalisée par oxydation directe des doubles liaisons, qui consiste à faire passer un courant d'oxygène à travers l'huile chauffée à 135°C (G., Soucek et al. "Spectroscopic investigation of blowing process of soybean oil", Surface Coatings International, Part B, Coatings Transactions. 2003, 86: 221-229). Le contrôle de l'oxydation n'est pas satisfaisant et de nombreux sous-produits sont formés tels que des peroxydes, aldéhydes, cétones, des scissions de chaînes, etc. Le seul avantage de ces polyols est leur faible coût de revient et leur synthèse réalisée en une seule étape, malgré les nombreux traitements appliqués au produit final (odeurs, indice d'acide élevé, couleur foncée, etc.).

Un catalyseur organométallique peut également être utilisé afin de mieux contrôler la réaction d'oxydation (WO2006/094227 ; WO2007/143135) en présence d'un oxydant.

Des polyols possédant des hydroxyles primaires peuvent être préparés par hydroformylation des insaturations (Guo et al., J. of Polymers and the Environment. 2002, 10: 49-52). Ce procédé fait intervenir une réaction entre du monoxyde de carbone et du dihydrogène, entraînant la formation d'un groupement aldéhyde qui est converti en hydroxyle par hydrogénation. Les catalyseurs à base de rhodium généralement utilisés sont très efficaces (conversions proches de 100%) mais aussi très coûteux. A l'inverse, les catalyseurs à base de cobalt sont bon marché mais moins efficaces. L'ozonolyse des doubles liaisons permet également d'obtenir des polyols ayant des groupements hydroxyles terminaux (Guo et al., J. of Polymer Sci., 2000, 38: 3900-3910). L'ozone passe à travers une solution d'huile végétale et d'éthylène glycol, en présence d'un catalyseur alcalin.

Une autre voie d'accès aux polyols consiste à réaliser une réaction préalable d'époxydation des insaturations. De nombreux travaux décrits dans la littérature décrivent l'époxydation de corps gras (Swern, et al., J. Am. Chem. Soc. 1944, 66, 1925-1927; Findley et al., J. Am. Chem. Soc. 1945, 67, 412-414; US 5 026 881; US 3 328 430; Petrovic' et al., Eur. J. Lipid Sci. Technol. 2002, 104: 293-299 et US 4 647 678). Petrovic a récemment démontré la possibilité de réaliser l'époxydation d'huile végétale par voie enzymatique (Vlc 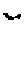 ek, T. et al., J. Amer. Oil Chem. Soc. 2006, 83: 247-252) ou catalysée par une résine échangeuse d'ions (Sinadinovic' -Fis 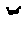 er et al., J. Amer. Oil Chem. Soc. 2001, 78: 725). Néanmoins, la voie la plus courante est l'utilisation d'un peracide formé in situ, généralement du peroxyde d'hydrogène en présence d'un acide carboxylique (le plus souvent de l'acide formique en quantité catalytique). La réaction est conduite entre 50 °C et 80°C pendant 1 à 4 heures.

La demande de brevet US 2007/0232816 décrit des polyols et leur utilisation comme monomères pour la préparation des polyuréthanes.

La présente invention a pour but de fournir un procédé de préparation de polyols à partir d'esters d'huile végétale permettant de s'affranchir des inconvénients susmentionnés.

La présente invention a également pour but de fournir un procédé qui, contrairement aux procédés de l'art antérieur qui concernent la transformation chimique à partir de triglycérides ayant des structures mal définies, consiste en une voie simple et efficace de modification chimique de monoesters ou de triglycérides pour l'obtention de précurseurs fonctionnels à fonctionnalité contrôlée.

La présente invention a pour but de fournir un procédé de préparation simple en deux étapes via des mono- ou des diesters d'huile végétale.

Un but de la présente invention est de fournir un procédé en deux étapes permettant l'accès à de nouveaux synthons, mono-esters ou diesters, tous au moins bifonctionnels (polyols) et possédant des structures bien définies.

La présente invention a également pour but de fournir un procédé de préparation de polyols présentant des fonctions hydroxyles primaires.

La présente invention concerne un procédé de préparation d'un polyol répondant à la formule générale (I') suivante :
dans laquelle : 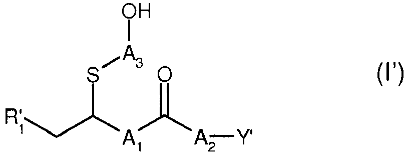
R'₁ représente un groupe alkyle, linéaire ou ramifié, comprenant de 1 à 14 atomes de carbone, ledit groupe alkyle pouvant éventuellement contenir un ou deux substituants latéraux -S-A₃-OH,
A₁ représente un radical alkylène divalent linéaire ou ramifié, comprenant de 2 à 14 atomes de carbone,
A₂ représente un radical -O-A₄-O-, A₄ représentant un radical alkylène divalent linéaire ou ramifié, comprenant de 1 à 20 atomes de carbone, de préférence de 1 à 10 atomes de carbone, le cas échéant comprenant un ou plusieurs substituants, notamment choisis dans le groupe constitué du radical phénylène et du radical de formule -(CH₂OCH₂)ₙ-, n représentant un nombre entier compris de 1 à 100, de préférence de 6 à 50, et préférentiellement égal à 6, 13 ou 45,
ou A₂ représente un radical de formule -(OCH₂CH₂)ₙ-O-, n étant tel que défini ci-dessus,
A₄ représentant de préférence un radical de formule -CH₂-A'₃-CH₂-, A'₃ représentant un groupe de formule -(CH₂OCH₂)ₙ-, n représentant un nombre entier compris de 1 à 100, de préférence de 6 à 50, et préférentiellement égal à 6, 13 ou 45, ou un radical phénylène,
A₃ représente un radical alkylène divalent linéaire ou ramifié, comprenant de 1 à 10 atomes de carbone, le cas échéant substitué,
Y' représente un atome d'hydrogène ou un groupe de formule (A') 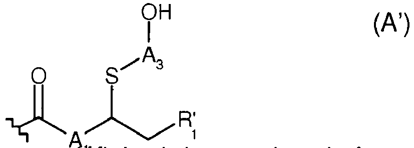 A₁, A₃ et R'₁ étant tels que définis ci-dessus dans la formule (I'), ledit procédé comprenant les étapes suivantes :
   a) une étape de transestérification d'un composé de formule (II') suivante : 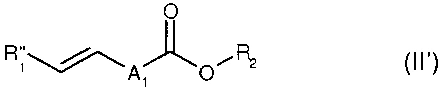 A₁ étant tel que défini ci-dessus dans la formule (I'),
      R"₁ représentant un groupe alkyle, linéaire ou ramifié, comprenant de 1 à 14 atomes de carbone, ledit groupe alkyle pouvant éventuellement contenir une ou deux doubles liaisons, et
      R₂ représentant un groupe alkyle, linéaire ou ramifié, comprenant de 1 à 10, de préférence de 1 à 6, atomes de carbone,
      avec un diol de formule (III) suivante : H-A₂-H (III)
      A₂ étant tel que défini ci-dessus dans la formule (I'),
      pour obtenir un composé de formule (IV') suivante : 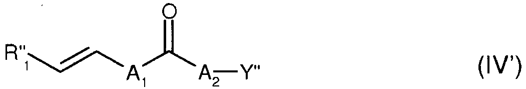 A₁, A₂ et R"₁ étant tels que définis ci-dessus, et
      Y" représentant un atome d'hydrogène ou un groupe de formule (A") 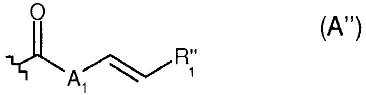 A₁ et R"₁ étant tels que définis ci-dessus,
   b) une étape de thiolisation du composé de formule (IV') susmentionnée pour obtenir un composé de formule (I') telle que définie ci-dessus, l'étape de thiolisation étant une étape de réaction du composé de formule (IV') susmentionnée avec un thiol de formule HS-A₃-OH, A₃ étant tel que défini ci-dessus dans la formule (I'), et
   c) une étape de récupération du composé de formule (I') telle que définie ci-dessus.

Ainsi, le procédé de l'invention consiste à synthétiser de nouveaux polymères à partir de mono-esters d'acides gras. Ces derniers sont généralement obtenus par exemple par transestérification des triglycérides avec un alcool court (R₂OH, R₂ étant tel que défini ci-dessus), de préférence avec du méthanol ou de l'éthanol.

Ces esters, et plus particulièrement les esters méthyliques ou éthyliques de tournesol ou de ricin, ont donc été utilisés comme 'synthons' de base dans la présente invention.

En utilisant une variété d'huile de tournesol dont la teneur en acide oléique est particulièrement élevée et en séparant par distillations fractionnées les différents esters méthyliques de l'huile de tournesol, on obtient des esters méthyliques d'acide oléique (une seule double liaison par chaîne grasse) de pureté élevée. C'est à partir de ce précurseur monofonctionnel qu'il est ensuite possible d'apporter un nombre choisi de groupes hydroxyles et ainsi de contrôler la fonctionnalité de ce 'synthon' monomère. Une structure bien définie de tels monomères est en effet indispensable à l'élaboration de matériaux polymères présentant des propriétés contrôlées et reproductibles. Les polymères désirés étant linéaires, on a par exemple cherché à créer des monomères au moins difonctionnels (di-OH) à partir d'esters méthyliques de tournesol oléique.

Les différentes réactions mises en jeu pour le procédé selon la présence invention sont (i) la transestérification du groupement ester par des diols permettant de greffer une première fonction hydroxyle primaire (étape a) susmentionnée), et (ii) une étape de thiolisation permettant de greffer une deuxième fonction hydroxyle primaire via une fonction thiol (étape b) susmentionnée).

Ainsi, la présente invention permet de préparer des diols répondant à l'une des deux formules suivantes : 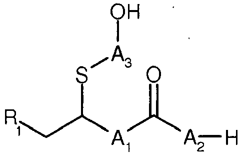 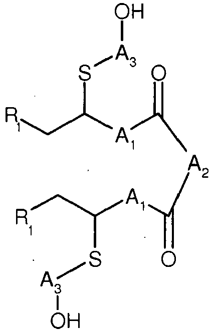 à savoir, d'une part, des diols non symétriques avec deux fonctions OH primaires, et, d'autre part, des diols symétriques avec deux fonctions OH primaires.

Selon la présente invention, les radicaux "alkyle" représentent des radicaux hydrocarbonés saturés, en chaîne droite ou ramifiée, comprenant de 1 à 14, de préférence de 1 à 10 atomes de carbone, et préférentiellement de 1 à 5 atomes de carbone (ils peuvent typiquement être représentés par la formule CₙH₂ₙ₊₁, n représentant le nombre d'atomes de carbone). On peut notamment citer, lorsqu'ils sont linéaires, les radicaux méthyle, éthyle, propyle, butyle, pentyle, hexyle, octyle, nonyle et décyle. On peut notamment citer, lorsqu'ils sont ramifiés ou substitués par un ou plusieurs radicaux alkyles, les radicaux isopropyle, tert-butyle, 2-éthylhexyle, 2-méthylbutyle, 2-méthylpentyle, 1-méthylpentyle et 3-méthylheptyle.

Selon la présente invention, les radicaux "alkylène" représentent des radicaux (également nommés alkylidènes) dérivés des alcanes dont les deux atomes d'hydrogène terminaux ont été supprimés. Lorsque lesdits radicaux alkylènes sont linéaires, ils peuvent être représentés par la formule -(CH₂)ₘ-, m correspondant au nombre d'atomes de carbone de l'alcane dont est issu le radical alkylène.

Selon un autre mode de réalisation, la présente invention concerne un procédé de préparation d'un diol répondant à la formule générale (I) suivante : 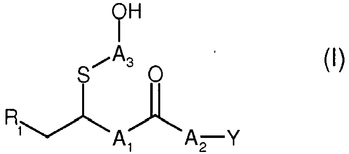 dans laquelle :
R₁ représente un groupe alkyle, linéaire ou ramifié, comprenant de 1 à 14 atomes de carbone,
A₁, A₂ et A₃ sont tels que définis ci-dessus dans la formule (I'), et
Y représente un atome d'hydrogène ou un groupe de formule (A) 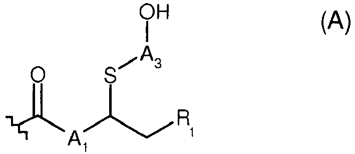
A₁, A₃ et R₁ étant tels que définis ci-dessus,
ledit procédé comprenant les étapes suivantes :
   a) une étape de transestérification d'un composé de formule (II) suivante : 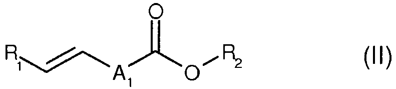 R₁ étant tel que défini ci-dessus,
      R₂ et A₁ étant tels que définis ci-dessus dans la formule (I'),
      avec un diol de formule (III) suivante : H-A₂-H (III)
      pour obtenir un composé de formule (IV) suivante : 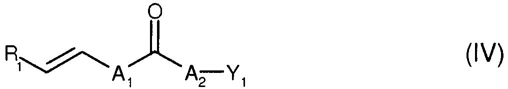 A₁, A₂ et R₁ étant tels que définis ci-dessus dans la formule (I),
      Y₁ représentant un atome d'hydrogène ou un groupe de formule (A₁) 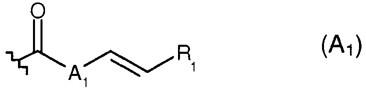 A₁ et R₁ étant tels que définis ci-dessus,
   b) une étape de thiolisation du composé de formule (IV) susmentionnée pour obtenir un composé de formule (I) telle que définie ci-dessus, et
   c) une étape de récupération du composé de formule (I) telle que définie ci-dessus.

La présente invention concerne également un procédé tel que défini ci-dessus pour la préparation d'un diol de formule (I-1) suivante : 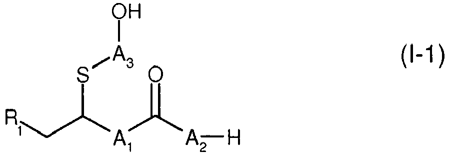 A₁, A₂, A₃ et R₁ étant tels que définis dans les formules (I) et (I').

Les composés de formule (I-1) sont des composés de formule (I) dans laquelle Y est un atome d'hydrogène. Ce diol comprend deux fonctions hydroxyles primaires.

La présente invention concerne également un procédé tel que défini ci-dessus pour la préparation d'un diol de formule (I-2) suivante : 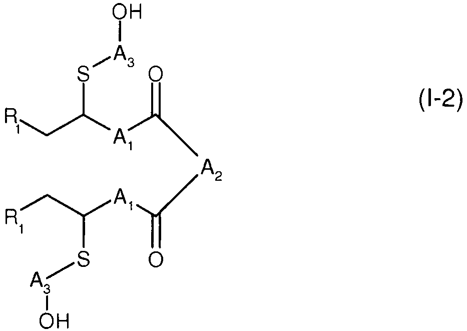 A₁, A₂, A₃ et R₁ étant tels que définis dans les formules (I) et (I').

Les composés de formule (I-2) sont des composés de formule (I) dans laquelle Y est un groupe de formule (A) telle que définie ci-dessus. Ce diol comprend deux fonctions hydroxyles primaires.

De préférence, l'étape a) est effectuée en présence d'un catalyseur choisi dans le groupe constitué de l'oxyde de magnésium, de l'acétate de zinc et du méthanolate de sodium.

Préférentiellement, l'étape a) du procédé selon l'invention est effectuée à une température comprise de 150 à 200°C sous flux d'azote.

Selon un mode de réalisation préféré, cette étape a) telle que définie ci-dessus est effectuée sans solvant, ce qui est très avantageux en termes d'écologie.

La présente invention concerne également un procédé de préparation d'un diol tel que défini ci-dessus, caractérisé en ce que le produit de formule (IV) obtenu à l'issue de l'étape a) est sous la forme d'un mélange de monoesters et de diesters, les monoesters répondant à la formule (IV-1) suivante : 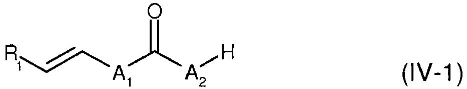 et les diesters répondant à la formule (IV-2) suivante : 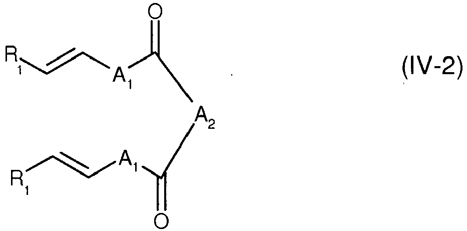 A₁, A₂ et R₁ étant tels que définis dans la formule (I).

Le monoester de formule (IV-1) correspond à un composé de formule (IV) dans laquelle Y₁ est H et le diester de formule (IV-2) correspond à un composé de formule (IV) dans laquelle Y₁ est un groupe de formule (A₁).

Selon un mode de réalisation préféré, l'étape de thiolisation du procédé de préparation tel que défini ci-dessus, est une réaction d'addition radicalaire en présence d'un amorceur radicalaire, notamment d'azobisisobutyronitrile (AIBN).

L'étape de thiolisation selon la présente invention consiste à faire réagir le composé obtenu à l'issue de l'étape a) avec un thiol de formule HS-A₃-OH, A₃ étant tel que défini ci-dessus.

De préférence, l'étape b) de thiolisation du procédé selon l'invention est effectuée en présence de toluène à une température comprise de 60°C à 80°C, et de préférence à 70°C.

Selon un mode de réalisation particulièrement préféré, l'étape b) consiste à faire réagir le composé de formule (IV) (ou (IV')) avec le 2-mercaptoéthanol (composé HS-A₃-OH où A₃ est un radical C₂H₄).

### Description détaillée des étapes du procédé de l'invention

### 1. Etape a) : réaction de transestérification des composés de formule (II') ou (II)

Dans le cadre du procédé de l'invention, cette transestérification s'effectue de préférence à partir d'un ester d'alcool léger (notamment méthanol ou éthanol...) de tournesol oléique (composé de formule (II) ou (II')) et d'un diol (composé de formule (III)) en présence notamment d'oxyde de magnésium comme catalyseur. Plusieurs synthèses sont réalisées avec différents diols afin de moduler les propriétés des monomères et donc des polymères en résultant. Des transestérifications ont donc été réalisées à partir de propanediol, d'hexanediol, de butanediol et de poly(oxyde d'éthylène)hydroxytéléchélique.

La réaction se déroule entre 150 °C et 200 °C sous flux d'azote. L'avancement de la réaction est suivi par différentes analyses et notamment la RMN (disparition du singulet du groupement méthyle). Selon les conditions de réaction, deux produits sont obtenus :
Si le diol utilisé est placé en grand excès, on obtient en majorité au moins 80%, voire 95%, de monoesters (ou dérivés de monoglycérides) possédant un groupement hydroxyle terminal. Cet alcool en bout de chaîne apporte alors une première fonctionnalité au monomère.

A l'inverse, si le diol est volontairement introduit en défaut, on obtient en majorité au moins 60%, voire 85%, de diesters (ou dérivés de diglycérides). Ce second précurseur possède alors exactement deux doubles liaisons par lesquelles seront introduits les groupements hydroxyles, permettant l'accès à des monomères de fonctionnalité égale à deux.

| | **Temps de réaction** | | **Rendement** | |
|---|---|---|---|---|
| **Alcools utilisés** | Synthèse de monoesters | Synthèse de diesters | Synthèse de monoesters | Synthèse de diesters |
| Propanediol | 10h | 15h | 80% | 62% |
| Hexanediol | 10h | 15h | 80% | 60% |
| Polyoxyde d'éthylène (M_{w}=300 g/mol) | 15h | 20h | 75% | 59% |
| Polyoxyde d'éthylène (M_{w}=600 g/mol) | 15h | 20h | 75% | 59% |

L'étape a) (avec R₁ = C₆H₁₃ ; A₁ = C₉H₁₈ ; R₂ = CH₃ ; A₂ = ORO) peut ainsi notamment être représentée par le schéma suivant :

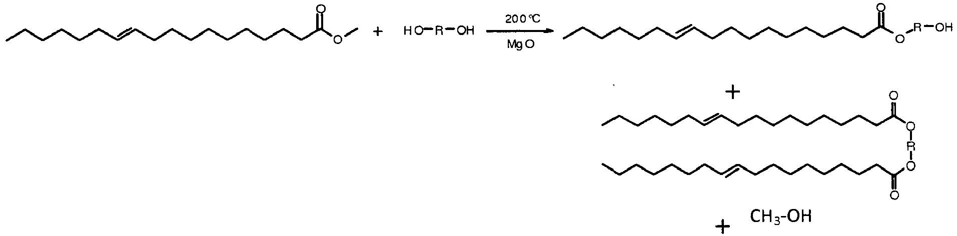

Les synthons préparés (correspondant aux composés de formule (IV)) sont par exemple purifiés sur colonne de silice avec un mélange 80/10/10 heptane/acétone/éther de pétrole pour le monoester et un mélange 80/20 heptane /éther de pétrole pour le diester. Les rendements après purification sont donnés dans le tableau ci-dessus.

A la fin de cette première étape on dispose de deux précurseurs : le premier est un monoester (composé de formule (IV-1)) possédant un groupement hydroxyle terminal et une double liaison sur la chaîne ; le second est un diester (composé de formule (IV-2)) possédant exactement deux doubles liaisons afin d'obtenir par la suite un polyol symétrique contenant deux groupements hydroxyles. La voie de synthèse mise en jeu est « propre » car elle fait appel à une catalyse hétérogène (oxyde de magnésium) et la synthèse se déroule sans solvant. La purification industrielle peut se faire par distillation sous vide poussé.

### 2. Etape b) : réaction d'addition radicalaire d'un thiol sur la double liaison

Il n'existe à ce jour aucune étude traitant de l'addition d'un thiol sur des monoesters ou diesters préalablement transesterifiés avec différents diols. Cette nouvelle voie permet l'obtention de nouveaux précurseurs possédant deux fonctions alcools primaires.

L'étape b) (avec R₁ = C₆H₁₃ ; A₁ = C₉H₁₈ ; A₂ = ORO et A₃ = C₂H₄) peut ainsi notamment être représentée par le schéma suivant :

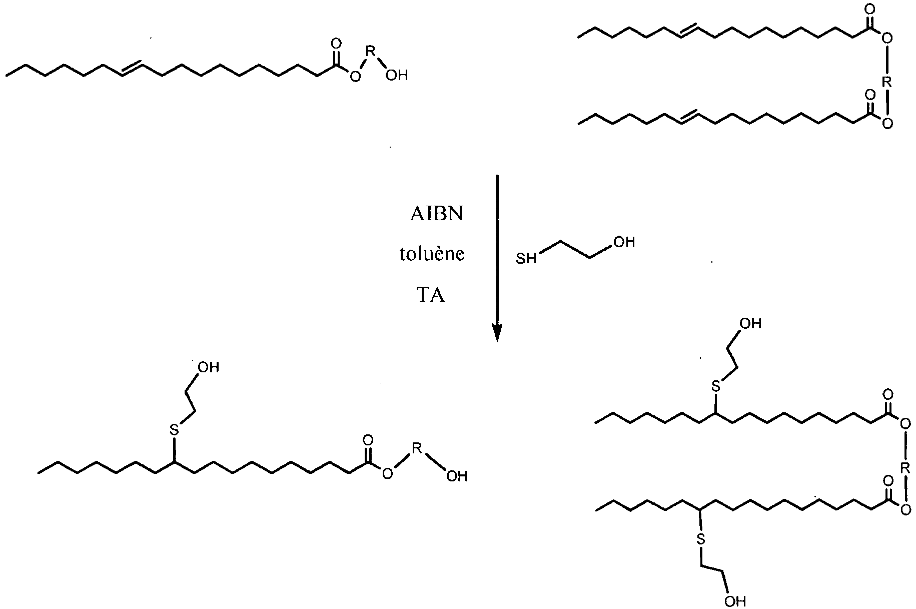

Les synthons décrits ci-dessus ont été préparés et purifiés sur colonne de silice avec un mélange toluène/acétate d'éthyle 60/40 pour la purification du monoester et un mélange toluène/acétate d'éthyle 70/ 30 pour le diester.

La présente invention concerne également des composés répondant à la formule générale (I') suivante : 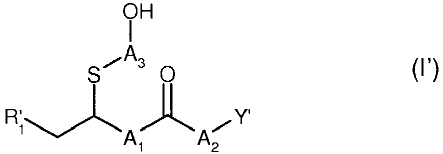 dans laquelle :
R'₁ représente un groupe alkyle, linéaire ou ramifié, comprenant de 1 à 14 atomes de carbone, ledit groupe alkyle pouvant éventuellement contenir un ou deux substituants latéraux -S-A₃-OH,
A₁ représente un radical alkylène divalent linéaire ou ramifié, comprenant de 2 à 14 atomes de carbone,
A₂ représente un radical -O-A₄-O-, A₄ représentant un radical alkylène divalent linéaire ou ramifié, comprenant de 1 à 20 atomes de carbone, de préférence de 1 à 10 atomes de carbone, le cas échéant comprenant un ou plusieurs substituants, notamment choisis dans le groupe constitué du radical phénylène et du radical de formule -(CH₂OCH₂)ₙ-, n représentant un nombre entier compris de 1 à 100, de préférence de 6 à 50, et préférentiellement égal à 6, 13 ou 45,
ou A₂ représente un radical de formule -(OCH₂CH₂)ₙ-O-, n étant tel que défini ci-dessus,
A₄ représentant de préférence un radical de formule -CH₂-A'₃-CH₂-, A'₃ représentant un groupe de formule -(CH₂OCH₂)ₙ-, n représentant un nombre entier compris de 1 à 100, de préférence de 6 à 50, et préférentiellement égal à 6, 13 ou 45, ou un radical phénylène,
A₃ représente un radical alkylène divalent linéaire ou ramifié, comprenant de 1 à 10 atomes de carbone, le cas échéant substitué, et
Y' représente un atome d'hydrogène ou un groupe de formule (A') 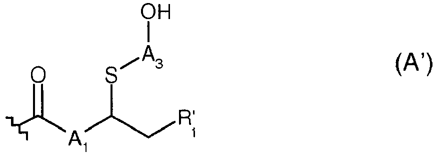
A₁, A₃ et R'₁ étant tels que définis ci-dessus dans la formule (I').

La présente invention concerne également des composés répondant à la formule générale (I) suivante : 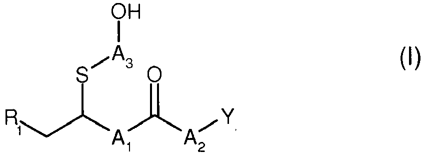 dans laquelle :
R₁ représente un groupe alkyle, linéaire ou ramifié, comprenant de 1 à 14 atomes de carbone,
A₁, A₂ et A₃ sont tels que définis dans la formule (I') ci-dessus, et
Y représente un atome d'hydrogène ou un groupe de formule (A) 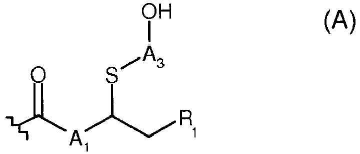
A₁, A₃ et R₁ étant tels que définis ci-dessus.

Des composés avantageux selon l'invention sont des composés répondant à la formule générale (I-1) suivante : 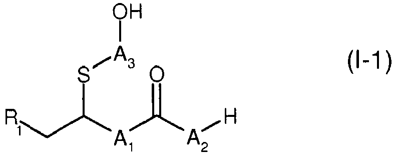 dans laquelle :
R₁, A₁, A₂, et A₃ sont tels que définis ci-dessus dans la formule (I').

Une famille de composés préférés selon l'invention est constituée de composés répondant à la formule générale (I-1-1) suivante : 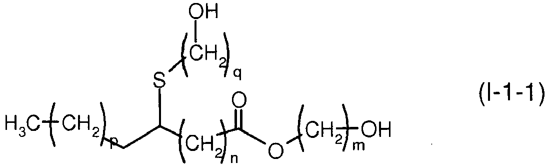 dans laquelle :
m, n, p et q sont des nombres entiers compris de 1 à 18.

Une autre famille de composés préférés selon l'invention est constituée de composés répondant à la formule générale (I-1-2) suivante :

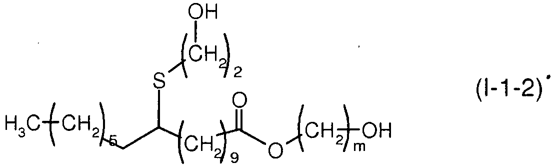

Parmi les composés préférés de l'invention, on peut citer notamment les composés répondant à la formule générale (I-2) suivante : 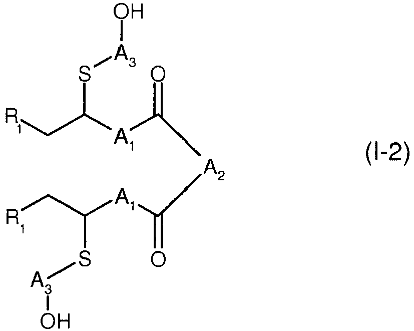 dans laquelle:
R₁, A₁, A₂ et A₃ sont tels que définis ci-dessus.

Une autre famille de composés préférés selon l'invention est constituée de composés répondant à la formule générale (I-2-1) suivante : 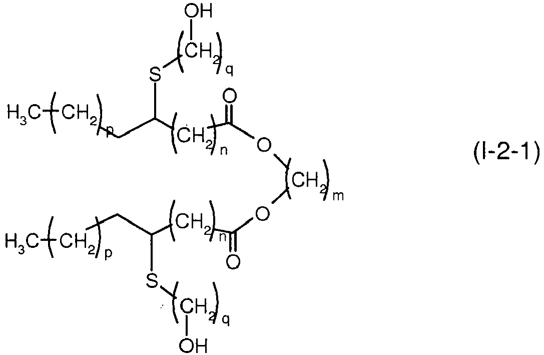 dans laquelle :
m, n, p et q sont des nombres entiers compris de 1 à 18.

Parmi les composés préférés de l'invention, on peut citer notamment les composés de formule générale (I-2-2) suivante :

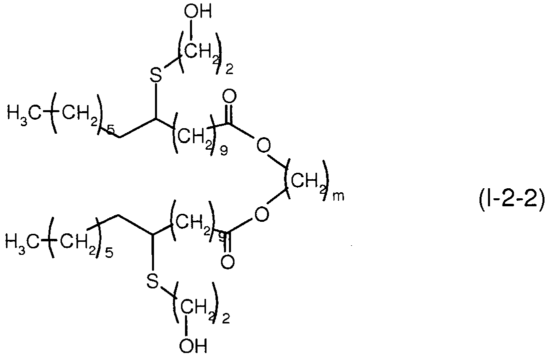

Les polyols selon la présente invention, notamment les diols, ont la spécificité d'être bien définis, avec deux groupements hydroxyles primaires. Les dérivés de diesters sont originaux en raison de leur symétrie et l'alcool utilisé pour la transestérification permet de varier la structure des synthons et ainsi les propriétés des polymères résultants.

Les diols obtenus par ces différentes méthodes peuvent alors être utilisés, entre autres, comme monomères. Leur pureté permet d'optimiser les propriétés des polymères obtenus.

Ainsi, des polyuréthanes ont ensuite été synthétisés par polymérisation en masse de ces polyols avec l'IPDI (ou par exemple également avec du MDI, du HMDI ou de l'HDI), à 60°C en présence de dibutyl dilaurate d'étain. La formation des polyuréthanes est confirmée par FTI R avec la disparition de la bande de vibration de l'isocyanate. La chromatographie d'exclusion stérique confirme des masses molaires comprises entre 14 000 et 50 000 g/mol. Ces monomères di-OH peuvent également être utilisés pour la synthèse d'autres polymères tels que des polyesters, polyéthers, polycarbonates, etc.

Les composés polyols selon la présente invention de formule (I) ou (I') sont notamment utilisés pour réagir avec des polyisocyanates.

Ainsi, ces composés peuvent être utilisés pour la préparation de mousses rigides, d'isolants électriques, de revêtements, d'adhésifs, de mousses flexibles (notamment dans le domaine de l'ameublement ou de l'automobile) ou de semelles de chaussures.

Plus exactement, les polyols selon la présente invention sont utilisés pour la préparation de mousses rigides en les faisant réagir avec des polyisocyanates en présence d'un catalyseur et d'un agent moussant (auxquels on peut également ajouter des surfactants, des colorants, des antioxydants, des conservateurs, des agents plastifiants, des agents de réticulation, des ignifuges, etc.).

De préférence, on peut préparer une telle mousse rigide en faisant réagir ensemble les constituants suivants : 60 g de polyisocyanate, 40 g de polyol, 1,2 g d'eau (agent moussant), 0,1-0,4 g de catalyseur et 1-4 g de surfactant.

Plus exactement, les polyols selon la présente invention sont utilisés pour la préparation d'isolants électriques en les faisant réagir avec des polyisocyanates en présence d'un agent anti-mousse et d'un agent séchant.

De préférence, un tel isolant électrique peut être préparé en faisant réagir ensemble 60 g de polyol, 29 g de polyisocyanate, 0,6 g d'agent anti-mousse et 3 g d'agent séchant, et éventuellement 60 g de charges (silice).

Plus exactement, les polyols selon la présente invention sont utilisés pour la préparation de revêtements en les faisant réagir avec des polyisocyanates. Par exemple, des revêtements sont préparés en utilisant les polyols et les polyisocyanate purs, ou en utilisant les polyols et les polyisocyanate avec solvants (on peut également ajouter des colorants, des pigments, des charges, des additifs rhéologiques, des antioxydants, des bactéricides, des fongicides, des inhibiteurs de corrosion, des catalyseurs ou des stabilisateurs UV).

Pour la préparation d'adhésifs selon la présente invention, on prévoit également d'utiliser les polyols de l'invention purs avec des polyisocyanates purs.

En ce qui concerne les mousses flexibles, de préférence, on utilise 60 g de polyol selon l'invention, 100 g d'isocyanate, 4.5 g d'eau (agent moussant), 0,12 g de catalyseur 1, 0,38 g de catalyseur 2 et 3 g de surfactant.

Enfin, une formulation spécifique selon l'invention pour la préparation de semelles de chaussures comprend 59 g d'isocyanate, 94,5 g de polyol selon l'invention, 4,1 g d'éthylène glycol et 1,4 g de catalyseur.

### PARTIE EXPÉRIMENTALE

### Exemple 1 : Préparation d'un diol de formule (I-2)

Le protocole décrit ci-après a été utilisé pour synthétiser des composés de formule (I-2), A₁ représentant un radical C₇H₁₄, A₃ représentant un radical C₂H₄ et R₁ représentant un groupe alkyle comprenant 9 atomes de carbone.

Dans la formule (I-2), A₂ peut représenter un radical choisi parmi les radicaux suivants : -OC₃H₆O-, -OC₄H₈O-, -OC₅H₁₀O-, -OC₆H₁₂O-, -OH₂C-(CH₂OCH₂)₆-CH₂O-, -OH₂C-(CH₂OCH₂)₁₃-CH₂O-, -OH₂C-(CH₂OCH₂)₄₅-CH₂O- ou -OH₂C-C₆H₄-CH₂O-.

### Etape de transestérification :

Les diesters sont issus de la transestérification d'un ester méthylique oléique et d'un diol (propanediol, butanediol, pentanediol, hexanediol, polyoxyde d'éthylène (300 g/mol, 600 g/mol et 2 000 g/mol)). La synthèse a fait intervenir 0,1 mol d'ester méthylique oléique et 0,05 mol de diol, en présence d'oxyde de magnésium MgO (catalyseur, 1 % en masse par rapport à la masse d'ester méthylique). Le milieu a été gardé sous agitation à 160°C, sous flux d'azote, pendant 7 heures. Le méthanol formé par la réaction a été enlevé du milieu réactionnel grâce à une trappe Dean Stark. La formation du diester a été suivie par RMN ¹H. Au bout de 7h, le milieu a été placé à 200°C sous vide dynamique pendant 1 h afin d'éliminer l'ester méthylique oléique et les diols résiduels. Le catalyseur a été éliminé par filtration.

Pour la synthèse du diester à partir d'ester méthylique et de 1,4-benzènediméthanol (A₂= -OH₂C-C₆H₄-CH₂O-), la température du milieu lors de la réaction était de 140°C afin de ne pas sublimer le 1,4-benzènediméthanol.

### Etape de thiolisation :

Un mélange de 10 mmol de diester précédemment synthétisé, 20 mmol de 2-mercaptoéthanol (HS-C₂H₄-OH) et d'azobisisobutyronitrile (catalyseur, 4% en masse par rapport à la masse de diester) est dissous dans 3 ml de toluène distillé. Le milieu est chauffé à 70°C, sous flux d'azote, pendant 8 h. L'avancée de la réaction est suivie par RMN ¹H. Lorsque la réaction est terminée, le 2-mercaptoéthanol résiduel est éliminé par distillation (180°C, sous vide dynamique).

### Exemple 2 : Préparation d'un diol de formule (I-1)

Le protocole décrit ci-après a été utilisé pour synthétiser des composés de formule (I-1), A₁ représentant un radical C₇H₁₄, A₃ représentant un radical C₂H₄ et R₁ représentant un groupe alkyle comprenant 9 atomes de carbone.

Dans la formule (I-1), A₂ peut représenter un radical choisi parmi les radicaux suivants : -OC₃H₆O-, -OC₄H₈O-, -OC₅H₁₀O-, -OC₆H₁₂O-, -OH₂C-(CH₂OCH₂)₆-CH₂O-, -OH₂C-(CH₂OCH₂)₁₃-CH₂O-, -OH₂C-(CH₂OCH₂)₄₅-CH₂O- ou -OH₂C-C₆H₄-CH₂O-.

Les protocoles de synthèse sont les mêmes que ceux indiqués pour l'exemple 1, exceptés pour l'étape de transestérification. La synthèse a fait intervenir 0,1 mol d'ester méthylique oléique et 1,5 mol de diol, afin de favoriser la formation de monoesters par rapport aux diesters.

### Exemple 3 : Préparation de polymères à partir de polyols de formule (I')

En appliquant les mêmes procédures que les exemples 1 et 2 susmentionnés, on a synthétisé le composé suivant : Synthèse de polymères issus du polyol

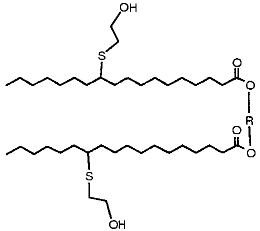

| | |
|---|---|
| Groupement R | C₃H₆ |
| Temps de réaction | 1h30 |
| M_{w} | 50 000 g/mol |
| IP | 1,6 |

### Exemple 4 : Préparation de diols de formule (I-2)

Le protocole décrit ci-dessus dans l'exemple 1 a été utilisé pour synthétiser les composés suivants :
- composé de formule (I-2), où A₁ représente un radical C₈H₁₆, A₂ représente un radical -OC₄H₈O-, A₃ représente un radical C₂H₄ et R₁ représente un groupe alkyle comprenant 8 atomes de carbone : 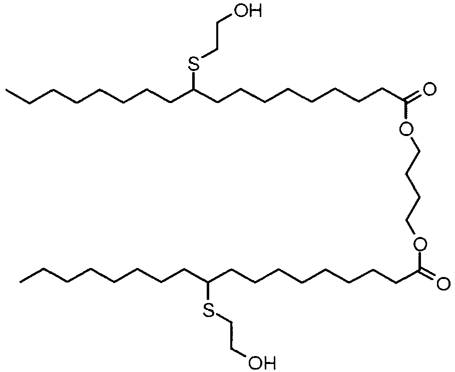
- composé de formule (I-2), où A₁ représente un radical C₈H₁₆, A₂ représente un radical -(OCH₂CH₂)₁₃-O-, A₃ représente un radical C₂H₄ et R₁ représente un groupe alkyle comprenant 8 atomes de carbone : 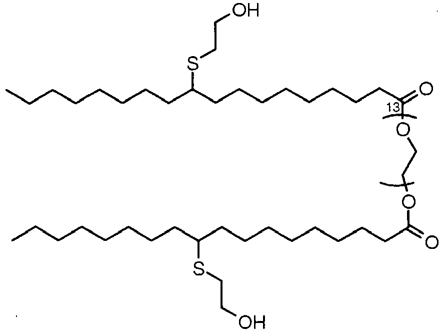
- composé de formule (I-2), où A₁ représente un radical C₈H₁₆, A₂ représente un radical -(OCH₂CH₂)₄₅-O-, A₃ représente un radical C₂H₄ et R₁ représente un groupe alkyle comprenant 8 atomes de carbone :

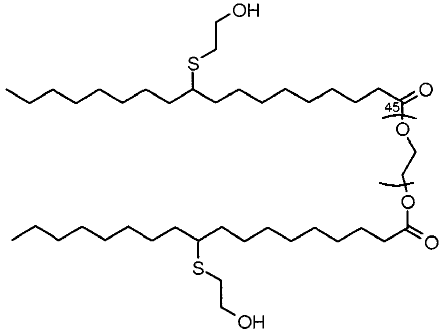

## Revendications

1. Procédé de préparation d'un polyol répondant à la formule générale (I') suivante : 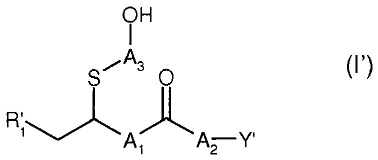 dans laquelle :
R'₁ représente un groupe alkyle, linéaire ou ramifié, comprenant de 1 à 14 atomes de carbone, ledit groupe alkyle pouvant éventuellement contenir un ou deux substituants latéraux, -S-A₃-OH,
A₁ représente un radical alkylène divalent linéaire ou ramifié, comprenant de 2 à 14 atomes de carbone,
A₂ représente un radical -O-A₄-O-, A₄ représentant un radical alkylène divalent linéaire ou ramifié, comprenant de 1 à 20 atomes de carbone, de préférence de 1 à 10 atomes de carbone, le cas échéant comprenant un ou plusieurs substituants, notamment choisis dans le groupe constitué du radical phénylène et du radical de formule -(CH₂OCH₂)ₙ-, n représentant un nombre entier compris de 1 à 100, de préférence de 6 à 50, et préférentiellement égal à 6, 13 ou 45, ou A₂ représente un radical de formule -(OCH₂CH₂)ₙ-O-, n étant tel que défini ci-dessus,
A₃ représente un radical alkylène divalent linéaire ou ramifié, comprenant de 1 à 10 atomes de carbone, le cas échéant substitué,
Y' représente un atome d'hydrogène ou un groupe de formule (A') 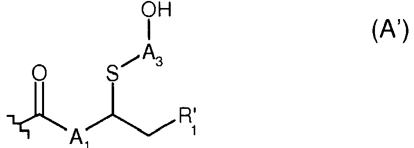 A₁, A₃ et R'₁ étant tels que définis ci-dessus dans la formule (I'),
ledit procédé comprenant les étapes suivantes :
a) une étape de transestérification d'un composé de formule (II') suivante : 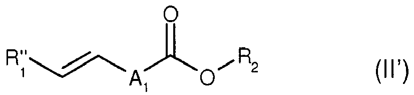 A₁ étant tel que défini ci-dessus dans la formule (I'),
R"₁ représentant un groupe alkyle, linéaire ou ramifié, comprenant de 1 à 14 atomes de carbone, ledit groupe alkyle pouvant éventuellement contenir une ou deux doubles liaisons, et
R₂ représentant un groupe alkyle, linéaire ou ramifié, comprenant de 1 à 10, de préférence de 1 à 6, atomes de carbone,
avec un diol de formule (III) suivante : H-A₂-H (III) A₂ étant tel que défini ci-dessus dans la formule (I'),
pour obtenir un composé de formule (IV') suivante : 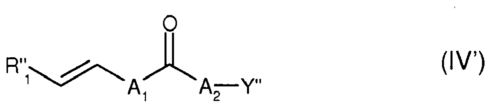 A₁, A₂ et R"₁ étant tels que définis ci-dessus, et
Y" représentant un atome d'hydrogène ou un groupe de formule (A") 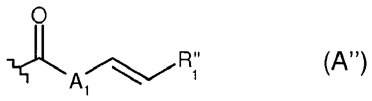 A₁ et R"₁ étant tels que définis ci-dessus,
b) une étape de thiolisation du composé de formule (IV') susmentionnée pour obtenir un composé de formule (I') telle que définie ci-dessus, et
c) une étape de récupération du composé de formule (I') telle que définie ci-dessus.

2. Procédé selon la revendication 1, pour la préparation d'un diol répondant à la formule générale (I) suivante : 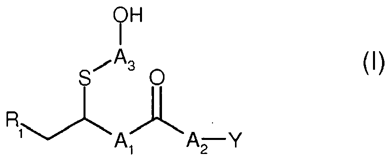 dans laquelle :
R₁ représente un groupe alkyle, linéaire ou ramifié, comprenant de 1 à 14 atomes de carbone,
A₁, A₂ et A₃ sont tels que définis dans la revendication 1, et
Y représente un atome d'hydrogène ou un groupe de formule (A) 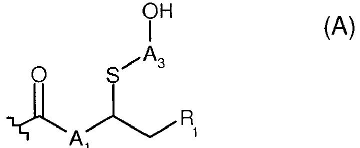
A₁, A₃ et R₁ étant tels que définis ci-dessus,
ledit procédé comprenant les étapes suivantes :
a) une étape de transestérification d'un composé de formule (II) suivante : 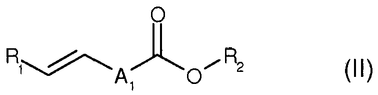 R₁ étant tel que défini ci-dessus dans la formule (I),
R₂ et A₁ étant tels que définis dans la revendication 1,
avec un diol de formule (III) suivante : H-A₂-H (III)
pour obtenir un composé de formule (IV) suivante : 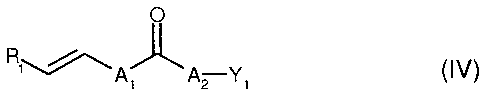 A₁, A₂ et R₁ étant tels que définis ci-dessus dans la formule (I), et
Y₁ représentant un atome d'hydrogène ou un groupe de formule (A₁) 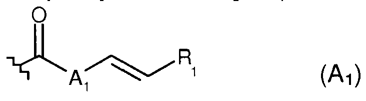 A₁ et R₁ étant tels que définis ci-dessus,
b) une étape de thiolisation du composé de formule (IV) susmentionnée pour obtenir un composé de formule (I) telle que définie ci-dessus, et
c) une étape de récupération du composé de formule (I) telle que définie ci-dessus.

3. Procédé de préparation d'un diol selon la revendication 2, **caractérisé en ce que** le diol répond à la formule (I-1) suivante : 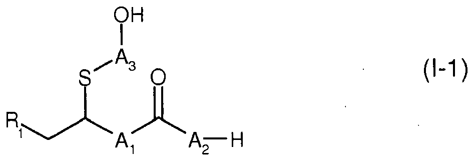 A₁, A₂, A₃ et R₁ étant tels que définis dans la revendication 2.

4. Procédé de préparation d'un diol selon la revendication 2, **caractérisé en ce que** le diol répond à la formule (I-2) suivante : 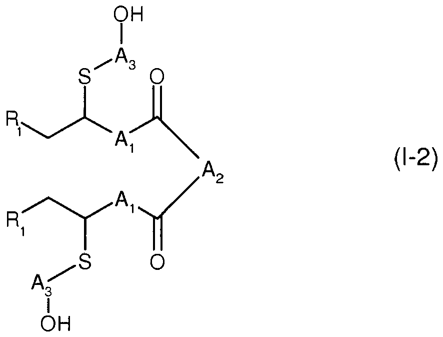 A₁, A₂, A₃ et R₁ étant tels que définis dans la revendication 2.

5. Procédé de préparation d'un diol selon l'une quelconque des revendications 1 à 4, dans lequel l'étape a) est effectuée en présence d'un catalyseur choisi dans le groupe constitué de l'oxyde de magnésium, de l'acétate de zinc et du méthanolate de sodium, notamment à une température comprise de 150 à 200°C sous flux d'azote.

6. Procédé de préparation d'un diol selon l'une quelconque des revendications 1 à 5, dans lequel l'étape de thiolisation est une réaction d'addition radicalaire en présence d'un amorceur radicalaire, notamment d'azobisisobutyronitrile (AIBN), cette étape étant de préférence effectuée en présence de toluène à une température comprise de 60°C à 80°C, et de préférence à 70°C.

7. Composé répondant à la formule générale (I') suivante : 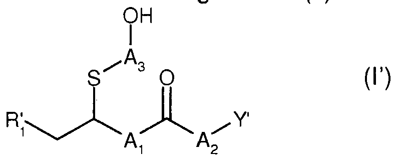 dans laquelle : R'₁ représente un groupe alkyle, linéaire ou ramifié, comprenant de 1 à 14 atomes de carbone, ledit groupe alkyle pouvant éventuellement contenir un ou deux substituants latéraux -S-A₃-OH,
A₁ représente un radical alkylène divalent linéaire ou ramifié, comprenant de 2 à 14 atomes de carbone,
A₂ représente un radical -O-A₄-O-, A₄ représentant un radical alkylène divalent linéaire ou ramifié, comprenant de 1 à 20 atomes de carbone, de préférence de 1 à 10 atomes de carbone, le cas échéant comprenant un ou plusieurs substituants, notamment choisis dans le groupe constitué du radical phénylène et du radical de formule -(CH₂OCH₂)ₙ-, n représentant un nombre entier compris de 1 à 100, de préférence de 6 à 50, et préférentiellement égal à 6, 13 ou 45, ou A₂ représente un radical de formule -(OCH₂CH₂)ₙ-O-, n étant tel que défini ci-dessus,
A₃ représente un radical alkylène divalent linéaire ou ramifié, comprenant de 1 à 10 atomes de carbone, le cas échéant substitué,
Y' représente un atome d'hydrogène ou un groupe de formule (A') 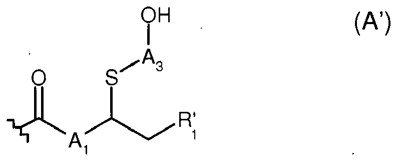 A₁, A₃ et R'₁ étant tels que définis ci-dessus dans la formule (I').

8. Composé selon la revendication 7, répondant à la formule générale (I) suivante : 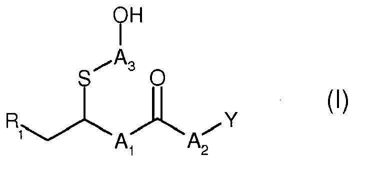 dans laquelle :
R₁ représente un groupe alkyle, linéaire ou ramifié, comprenant de 1 à 14 atomes de carbone,
A₁, A₂ et A₃ sont tels que définis dans la revendication 7, et
Y représente un atome d'hydrogène ou un groupe de formule (A) 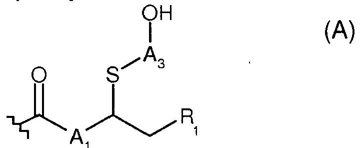 A₁, A₃ et R₁ étant tels que définis ci-dessus.

9. Composé selon la revendication 8, répondant à la formule générale (I-1) suivante : 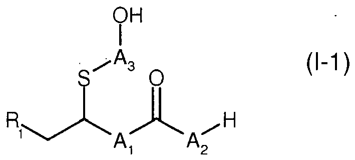 dans laquelle :
R₁, A₁, A₂, et A₃ sont tels que définis dans la revendication 8.

10. Composé selon la revendication 8, répondant à la formule générale (I-2) suivante : 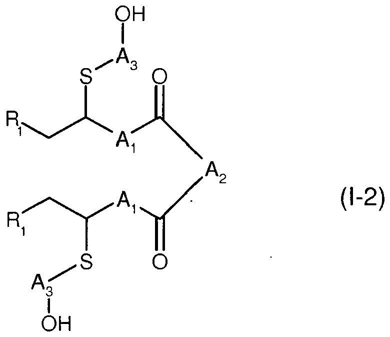 dans laquelle :
R₁, A₁, A₂ et A₃ sont tels que définis dans la revendication 8.

11. Composé selon la revendication 10, répondant à la formule générale (I-2-1) suivante : 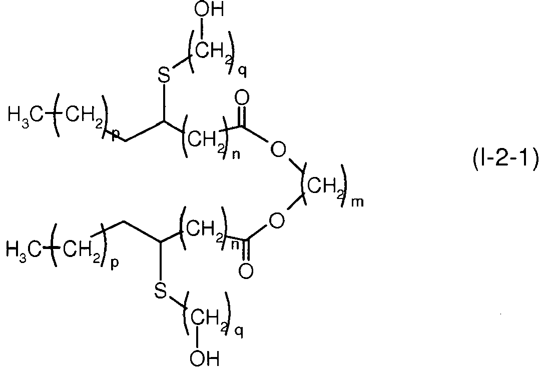 dans laquelle :
m, n, p et q sont des nombres entiers compris de 1 à 18.

## Patentansprüche

1. Verfahren zur Herstellung eines Polyols, das der folgenden allgemeinen Formel entspricht: 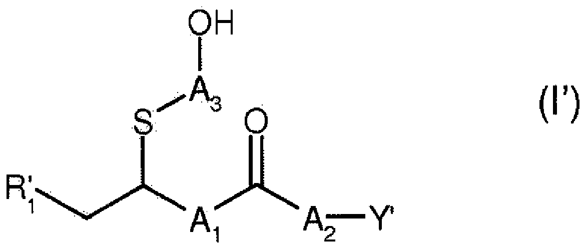 in der:
R'₁ eine lineare oder verzweigte Alkylgruppe darstellt, die 1 bis 14 Kohlenstoffatome enthält, wobei die Alkylgruppe gegebenenfalls einen oder zwei Seitensubstituenten -S-A₃-OH enthalten kann,
A₁ einen linearen oder verzweigten divalenten Alkylenrest darstellt, der 2 bis 14 Kohlenstoffatome enthält,
A₂ einen -O-A₄-O-Rest darstellt, wobei A₄ einen linearen oder verzweigten divalenten Alkylenrest darstellt, der 1 bis 20 Kohlenstoffatome, vorzugsweise 1 bis 10 Kohlenstoffatome, enthält, gegebenenfalls einen oder mehrere Substituenten enthält, die insbesondere ausgewählt sind aus der Gruppe bestehend aus dem Phenylenrest und dem Rest der Formel -(CH₂OCH₂)ₙ-, wobei n eine ganze Zahl zwischen 1 und 100, vorzugsweise zwischen 6 und 50 und besonders bevorzugt gleich 6, 13, oder 45, darstellt oder A₂ einen Rest der Formel - (OCH₂CH₂)ₙ-O- darstellt, wobei n so definiert wird wie oben,
A₃ einen divalenten linearen oder verzweigten Alkylenrest darstellt, der 1 bis 10 Kohlenstoffatome enthält, gegebenenfalls substituiert,
Y' ein Wasserstoffatom oder eine Gruppe der Formel (A') darstellt 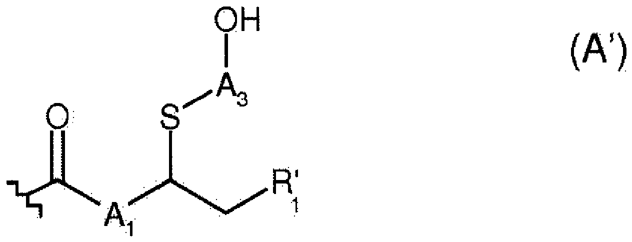
wobei A₁, A₃ und R'₁ so definiert werden wie oben in der Formel (I'),
wobei das Verfahren die folgenden Schritte umfasst:
a) einen Schritt der Umesterung einer Verbindung der folgenden Formel (II'): 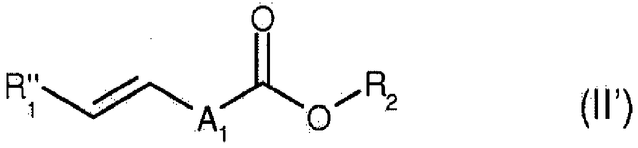 wobei A₁ so definiert ist wie in der Formel (I'),
R"₁ eine lineare oder verzweigte Alkylgruppe darstellt, die 1 bis 14 Kohlenstoffatome umfasst, wobei die Alkylgruppe gegebenenfalls eine oder zwei Doppelbindungen enthalten kann, und
R₂ eine lineare oder verzweigte Alkylgruppe darstellt, die 1 bis 10, vorzugsweise 1 bis 6 Kohlenstoffatome, umfasst,
mit einem Diol der folgenden Formel (III): H-A₂-H (III)
wobei A₂ so definiert ist wie oben in der Formel (I'),
um eine Verbindung der folgenden Formel (IV') zu erhalten: 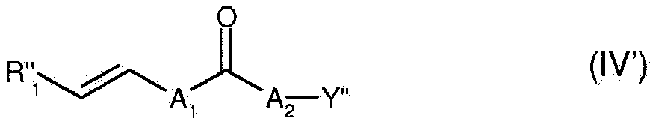 wobei A₁, A₂ und R"₁ so definiert sind wie oben und
Y" ein Wasserstoffatom oder eine Gruppe der Formel (A") darstellt, 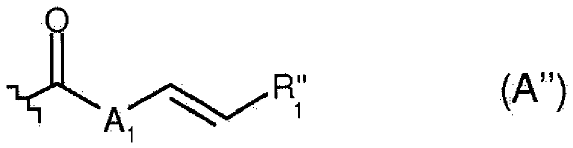 wobei A₁ und R"₁ so definiert sind wie oben,
b) einen Schritt der Thiolisierung einer Verbindung der oben erwähnten Formel (IV'), um eine Verbindung der Formel (I'), wie sie oben definiert ist, zu erhalten, und
c) einen Schritt der Rückgewinnung der Verbindung der Formel (I'), wie sie oben definiert ist.

2. Verfahren nach Anspruch 1 für die Herstellung eines Diols, das der folgenden allgemeinen Formel (I) entspricht: 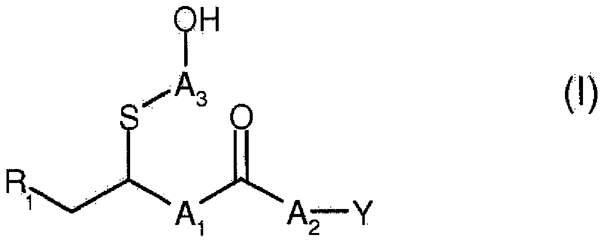 in der:
R₁ eine lineare oder verzweigte Alkylgruppe darstellt, die 1 bis 14 Kohlenstoffatome enthält,
A₁, A₂ und A₃ so definiert sind wie in dem Anspruch 1 und
Y ein Wasserstoffatom oder eine Gruppe der Formel (A) darstellt, 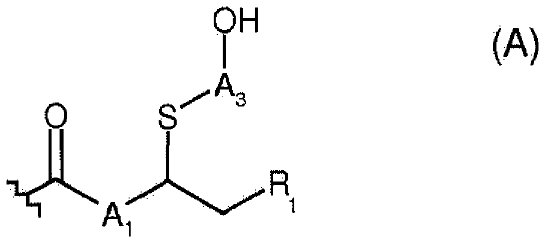
wobei A₁, A₃ und R₁ so definiert sind wie oben,
wobei das Verfahren die folgenden Schritte enthält:
a) einen Schritt der Umesterung einer Verbindung der folgenden Formel (II): 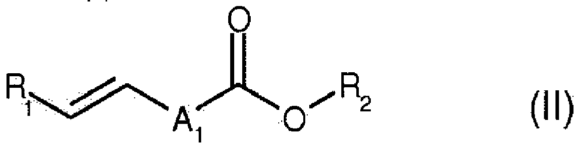 wobei R₁ so definiert ist wie in der obigen Formel (I),
R₂ und A₁ so definiert sind wie in dem Anspruch 1,
mit einem Diol der folgenden Formel (III): H-A₂-H (III)
um eine Verbindung der folgenden Formel (IV) zu erhalten: 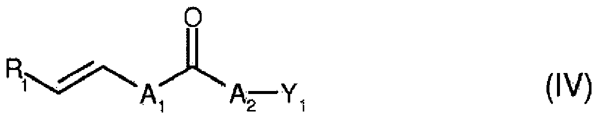 wobei A₁, A₂ und R₁ so definiert sind wie oben in der Formel (I) und
Y₁ ein Wasserstoffatom oder eine Gruppe der Formel (A₁) darstellt, 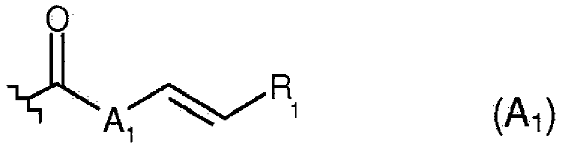 wobei A₁ und R₁ so definiert sind wie oben,
b) einen Schritt der Thiolisierung der Verbindung der oben erwähnten Formel (IV), um eine Verbindung der Formel (I), wie sie oben definiert ist, zu erhalten, und
c) einen Schritt der Rückgewinnung der Verbindung der Formel (I), wie sie oben definiert ist.

3. Verfahren zur Herstellung eines Diols nach Anspruch 2, **dadurch gekennzeichnet, dass** das Diol der folgenden Formel (I-1) entspricht: 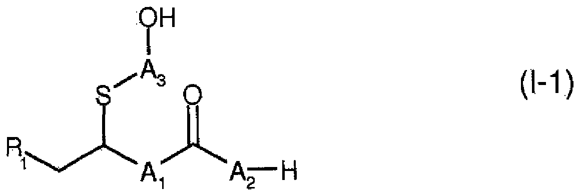 wobei A₁, A₂, A₃ und R₁ so definiert sind wie im Anspruch 2.

4. Verfahren zur Herstellung eines Diols nach Anspruch 2, **dadurch gekennzeichnet, dass** das Diol der folgenden Formel (I-2) entspricht: 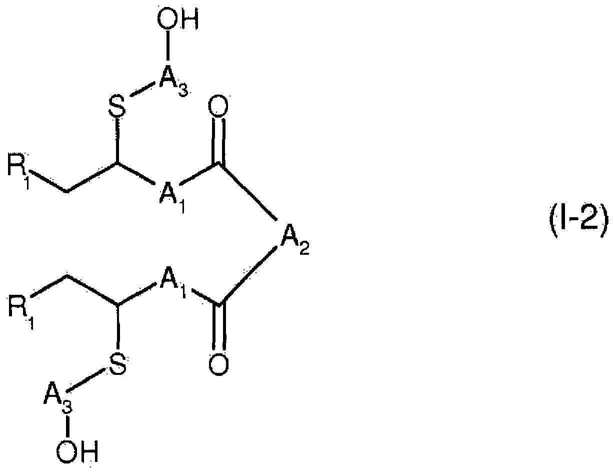 wobei A₁, A₂, A₃ und R₁ so definiert sind wie in dem Anspruch 2.

5. Verfahren zur Herstellung eines Diols nach einem beliebigen der Ansprüche 1 bis 4, bei dem der Schritt a) in Anwesenheit eines Katalysators durchgeführt wird, der ausgewählt ist aus der Gruppe bestehend aus Magnesiumoxid, Zinkacetat und Natriummethanolat, insbesondere bei einer Temperatur zwischen 150°C und 200°C bei einem Stickstoffstrom.

6. Verfahren zur Herstellung eines Diols nach einem beliebigen der Ansprüche 1 bis 5, bei dem der Schritt der Thiolisierung eine radikalische Additionsreaktion in Gegenwart eines radikalischen Initiators, insbesondere des Azobisisobutyronitrils (AIBN), ist, wobei dieser Schritt vorzugsweise in Gegenwart von Toluol bei einer Temperatur zwischen 60°C und 80°C und vorzugweise bei 70°C durchgeführt wird.

7. Verbindung, die der folgenden allgemeinen Formel (I') entspricht: 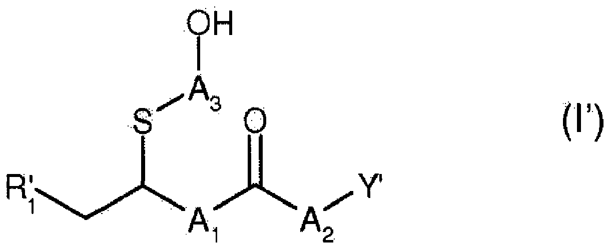 in der:
R'₁ eine lineare oder verzweigte Alkylgruppe darstellt, die 1 bis 14 Kohlenstoffatome enthält, wobei die Alkylgruppe gegebenenfalls einen oder zwei Seitensubstituenten -S-A₃-OH enthalten kann,
A₁ einen linearen oder verzweigten divalenten Alkylenrest darstellt, der 2 bis 14 Kohlenstoffatome enthält,
A₂ einen -O-A₄-O-Rest darstellt, wobei A₄ einen linearen oder verzweigten divalenten Alkylenrest darstellt, der 1 bis 20 Kohlenstoffatome, vorzugsweise 1 bis 10 Kohlenstoffatome, enthält, gegebenenfalls einen oder mehrere Substituenten enthält, die insbesondere ausgewählt sind aus der Gruppe bestehend aus dem Phenylenrest und dem Rest der Formel -(CH₂OCH₂)ₙ-, wobei n eine ganze Zahl zwischen 1 und 100, vorzugsweise zwischen 6 und 50 und besonders bevorzugt gleich 6, 13, oder 45, darstellt oder A₂ einen Rest der Formel - (OCH₂CH₂)ₙ-O- darstellt, wobei n so definiert wird wie oben,
A₃ einen divalenten linearen oder verzweigten Alkylenrest darstellt, der 1 bis 10 Kohlenstoffatome enthält, gegebenenfalls substituiert,
Y' ein Wasserstoffatom oder eine Gruppe der Formel (A') darstellt 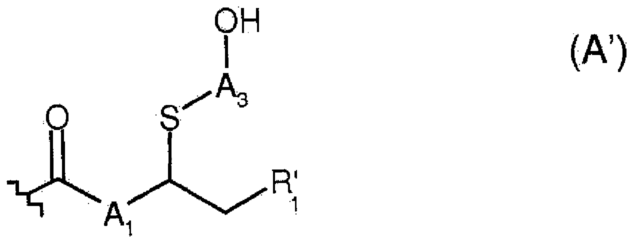
wobei A₁, A₃ und R'₁ so definiert werden wie oben in der Formel (I').

8. Verbindung nach Anspruch 7, die der folgenden allgemeinen Formel (I) entspricht: 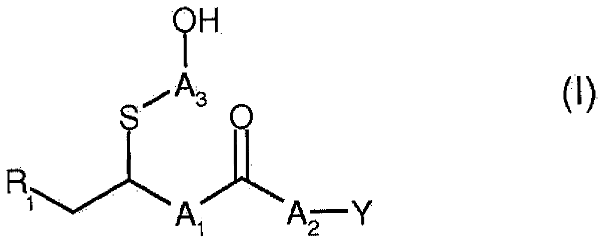 in der:
R₁ eine lineare oder verzweigte Alkylgruppe darstellt, die 1 bis 14 Kohlenstoffatome enthält,
A₁, A₂ und A₃ so definiert sind wie in dem Anspruch 7 und
Y ein Wasserstoffatom oder eine Gruppe der Formel (A) darstellt, 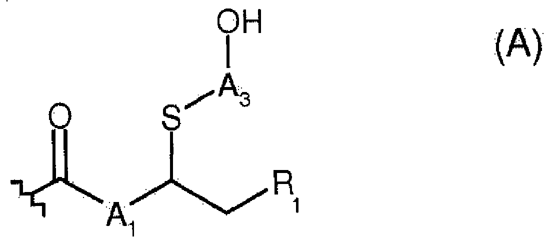
wobei A₁, A₃ und R₁ so definiert sind wie oben.

9. Verbindung nach Anspruch 8, die der allgemeinen folgenden Formel (I-1) entspricht: 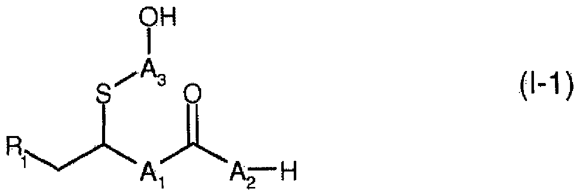 in der:
R₁, A₁, A₂ und A₃ so definiert sind wie im Anspruch 8.

10. Verbindung nach Anspruch 8, die der folgenden allgemeinen Formel (I-2) entspricht: 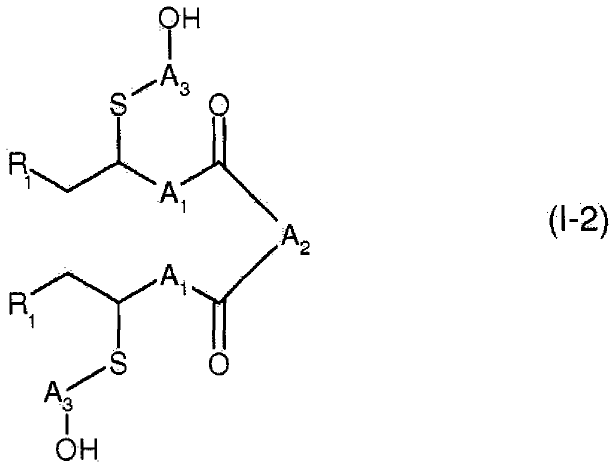 in der:
R₁, A₁, A₂ und A₃ so definiert sind wie im Anspruch 8.

11. Verbindung nach Anspruch 10, die der folgenden allgemeinen Formel (I-2-1) entspricht: 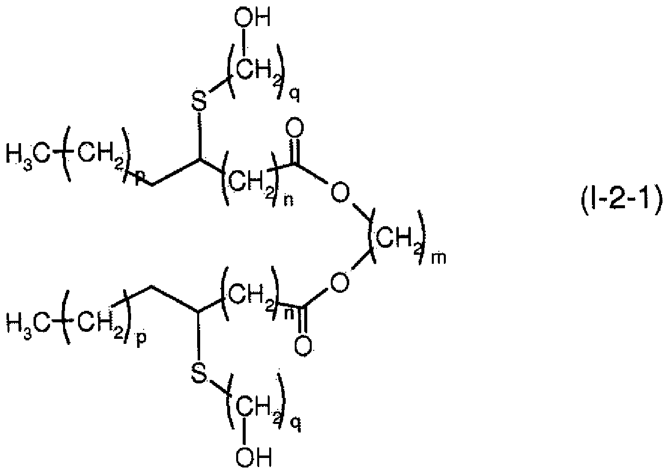 in der:
m, n, p und q ganze Zahlen zwischen 1 und 18 sind.

## Claims

1. A method for preparing a polyol meeting the following general formula (I') :
wherein:
R'₁ is a straight-chain or branched alkyl group comprising 1 to 14 carbon atoms, the said alkyl group optionally containing one or more side substituents -S-A₃-OH,
A₁ is a divalent alkylene radical, straight-chain or branched, comprising 2 to 14 carbon atoms,
A₂ is a -O-A₄-O- radical, A₄ is a divalent alkylene radical, straight-chain or branched, comprising 1 to 20 carbon atoms" preferably 1 to 10 carbon atoms, optionally comprising one or more substituents chosen in particular from the group formed of the phenylene radical and the radical of formula (CH₂OCH₂)ₙ-, n representing an integer of between 1 to 100, preferably 6 to 50 and preferably it is 6, 13 or 45, or A₂ is a radical of formula -(OCH₂CH₂)ₙ-O-, n being such as defined above,
A₃ is a divalent alkylene radical, straight-chain or branched, comprising 1 to 10 carbon atoms, optionally substituted,
Y' is a hydrogen atom or a group of formula (A') 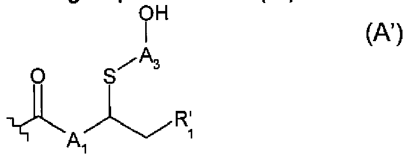
A₁, A₃ and R'₁ being such as defined above in formula (I'),
the said method comprising the following steps:
a) a transesterification step of a compound of following formula (II'): 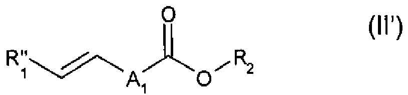 A₁ being such as defined above in formula (I'), 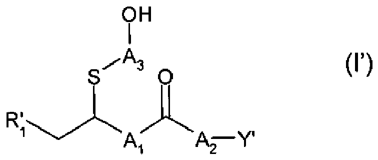 R"₁ representing a straight-chain or branched alkyl group comprising 1 to 14 carbon atoms, the said alkyl group optionally containing one or two double bonds,and
R₂ representing a straight-chain or branched alkyl group comprising 1 to 10, preferably 1 to 6 carbon atoms,
with a diol of following formula (III) : H-A₂-H (III)
A₂ being such as defined above in formula (I'),
to obtain a compound of following formula (IV'): 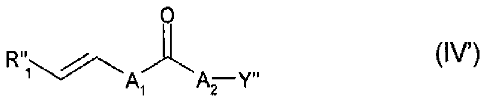 A₁, A₂ and R"₁ being such as defined above, and,
Y" representing a hydrogen atom or a group of formula (A") 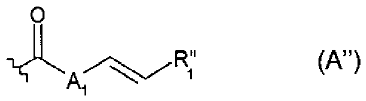 A₁ and R"₁ being such as defined above,
b) a thiolation step of the compound of above-mentioned formula (IV') to obtain a compound of formula (I') such as defined above, and
c) a step to collect the compound of formula (I') such as defined above.

2. The method according to claim 1, to prepare a diol meeting the following general formula (I): 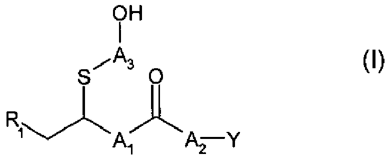 wherein:
R₁ is a straight-chain or branched alkyl group comprising 1 to 14 carbon atoms,
A₁, A₂ and A₃ are such as defined in claim 1, and
Y is a hydrogen atom or a group of formula (A) 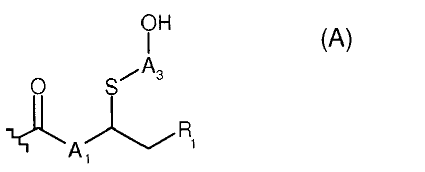
A₁, A₃ and R₁ being such as defined above,
the said method comprising the following steps:
a) a transesterification step of a compound of the following formula (II): 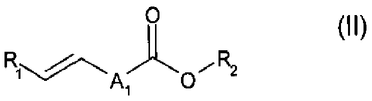 R₁ being such as defined above in formula (I),
R₂ and A₁ being such as defined in claim 1,
with a diol of following formula (III) : H-A₂-H (III)
to obtain a compound of compound of following formula (IV): 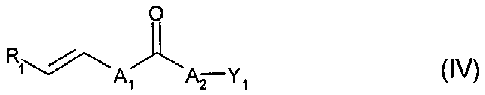 A₁, A₂ and R₁ being such as defined above in formula (I), and
Y₁ representing a hydrogen atom or a group of formula (A₁) 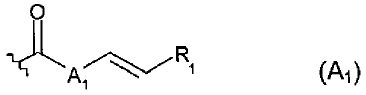 A₁ and R₁ being such as defined above,
b) a thiolation step of the compound of above-mentioned formula (IV) to obtain a compound of formula (I) such as defined above, and
c) a step to collect the compound of formula (I) such as defined above.

3. The method for preparing a diol according to claim 2, **characterized in that** the diol meets the following formula (I-1): 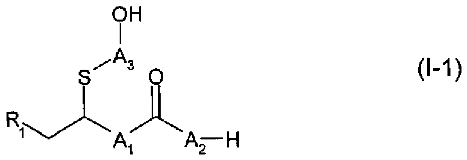 A₁, A₂, A₃ and R₁ being such as defined in claim 2.

4. The method for preparing a diol according to claim 2, **characterized in that** the diol meets the following formula (I-2): 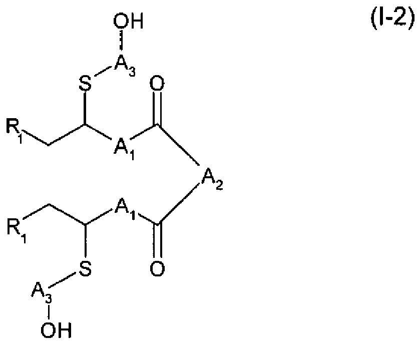 A₁, A₂, A₃ and R₁ being such as defined in claim 2.

5. The method for preparing a diol according to any of claims 1 to 4, wherein step a) is conducted in the presence of a catalyst chosen from the group formed of magnesium oxide, zinc acetate and sodium methanolate, in particular at a temperature of between 150 and 200°C under a stream of nitrogen.

6. The method for preparing a diol according to any of claims 1 to 5, wherein the thiolation step is a radical addition reaction in the presence of a radical initiator, azobisisobutyronitrile (AIBN) in particular, this step preferably being conducted in the presence of toluene at a temperature of between 60°C and 80°C, preferably at 70°C.

7. A compound meeting the following general formula (I'): 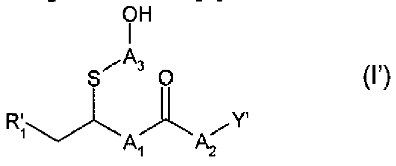 wherein:
R'₁ is a straight-chain or branched alkyl group, comprising 1 to 14 carbon atoms, the said alkyl group optionally containing one or two side substituents - S-A₃-OH,
A₁ is a divalent straight-chain or branched alkylene radical, comprising 2 to 14 carbon atoms,
A₂ is a radical -O-A₄-O-, A₄ representing a divalent alkylene radical, straight-chain or branched, comprising 1 to 20 carbon atoms, preferably 1 to 10 carbon atoms, optionally comprising one or more substituents, chosen in particular from the group formed of the phenylene radical or the radical of formula -(CH₂OCH₂)ₙ-, n representing an integer of between 1 and 100 preferably 6 to 50, and is preferably 6, 13 or 45, or A₂ representing a radical of formula -(OCH₂CH₂)ₙ-O-, n being such as defined above,
A₃ is a divalent alkylene radical, straight-chain or branched, comprising 1 to 10 carbon atoms, optionally substituted,
Y' is a hydrogen atom or a group of formula (A') 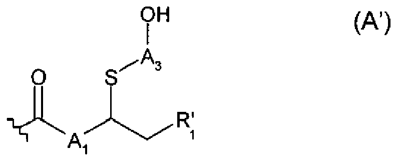
A₁, A₃ and R'₁ being such as defined above in formula (I').

8. The compound according to claim 7 meeting the following general formula (I): 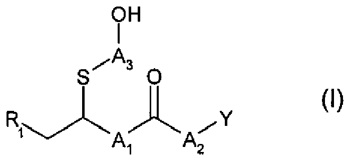 wherein:
R₁ is a straight-chain or branched alkyl group, comprising 1 to 14 carbon atoms,
A₁, A₂ and A₃ are such as defined in claim 7, and
Y is a hydrogen atom or a group of formula (A) 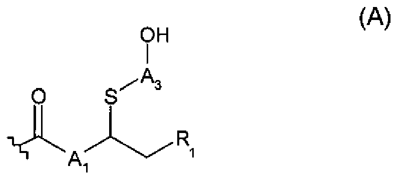
A₁, A₃ and R₁ being such as defined above.

9. The compound according to claim 8 meeting the following general formula (I-1): 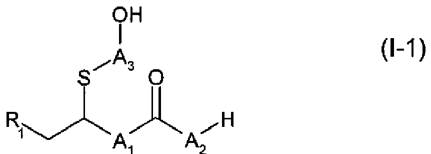 wherein:
R₁, A₁, A₂, and A₃ are such as defined in claim 8.

10. The compound according to claim 8 meeting the following general formula (I-2): 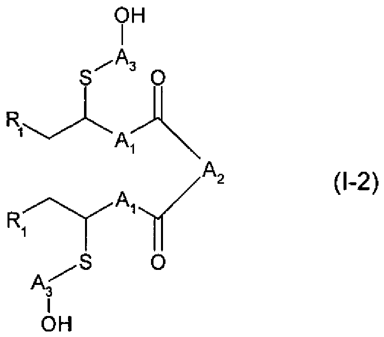 wherein:
R₁, A₁, A₂ and A₃ are such as defined in claim 8.

11. The compound according to claim 10 meeting the following general formula (I-2-1): 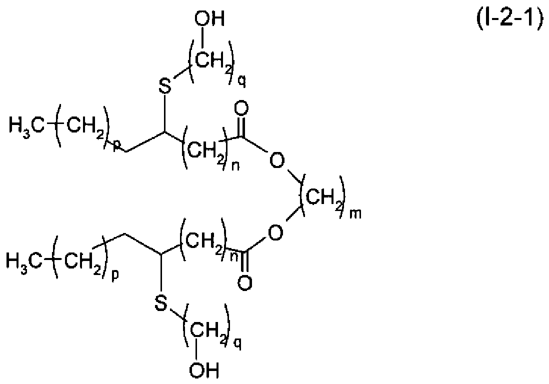 wherein:
m, n, p and q are integers of between 1 and 18.
